Europäisches Patentamt

European Patent Office

Office européen des brevets

(11) Veröffentlichungsnummer: **0 114 161**
**B1**

(12) **EUROPÄISCHE PATENTSCHRIFT**

(45) Veröffentlichungstag der Patentschrift:
07.06.89

(51) Int. Cl.$^4$: **C 12 P 7/06**

(21) Anmeldenummer: 84890008.0

(22) Anmeldetag: 10.01.84

(54) Verfahren zur Gewinnung von Äthanol aus vergärbaren Zuckerlösungen.

(30) Priorität: 13.01.83 AT 108/83
13.01.83 AT 106/83

(43) Veröffentlichungstag der Anmeldung:
25.07.84 Patentblatt 84/30

(45) Bekanntmachung des Hinweises auf die Patenterteilung: 07.06.89 Patentblatt 89/23

(84) Benannte Vertragsstaaten:
AT BE CH DE FR GB IT LI LU NL SE

(56) Entgegenhaltungen:
WO-A-81/031 82
GB-A- 2 054 645
GB-A- 2 066 843
GB-A- 2 075 053
US-A- 2 440 925
US-A- 4 337 123
US-A- 4 359 533
BIOTECHNOLOGY AND BIOENGINEERING, Band XIX, August 1977, Seiten 1125-1143, John Wiley & Sons Inc., New York, US; G.R. CYSEWSKI et al.: "Rapid ethanol fermentations using vacuum and cell recycle"

(73) Patentinhaber: VOEST-ALPINE Aktiengesellschaft
Friedrichstrasse 4
A-1011 Wien (AT)

(72) Erfinder: Cvitas, Vilim, Dipl.-Ing.
Voltastrasse 29
A-4040 Linz (AT)
Erfinder: Faltejsek, Karl, Dipl.-Ing.
Lüfteneggerstrasse 6
A-4020 Linz (AT)
Erfinder: Hanke, Reinhart, Dipl.-Ing.
Annaberggasse 2
A-8700 Leoben (AT)
Erfinder: Treso, Bertalan
Kunigundenweg 11a
A-8707 Leoben (AT)

(74) Vertreter: Haffner, Thomas M., Dr. et al
Patentanwaltskanzlei Dipl.-Ing. Adolf Kretschmer Dr.
Thomas M. Haffner Schottengasse 3a
A-1014 Wien (AT)

**Beschreibung**

Die Erfindung bezieht sich auf ein Verfahren zur Gewinnung von Äthanol aus vergärbaren Zuckerlösungen, bei welchem die Zuckerlösung vor dem Einbringen in einen Gärbehälter mit Hefe versetzt wird, und der durch Gärung gebildete Alkohol durch Anwendung von unteratmosphärischem Druck über die Gasphase aus dem Gärbehälter abgezogen wird. Aus der EP-A-0 044 428 ist es bereits bekannt, zur Herstellung von Alkohol aus Stärke oder stärkehaltigen Rohstoffen, eine Schlempe zu gewinnen, welche durch Zerkleinern, thermischen Aufschluß und Verzuckerung der stärkehaltigen Rohstoffe gebildet wird. Es sind eine Reihe von zuckerhaltigen Rohstoffen, bekannt, aus welchen unmittelbar Zuckerlösungen auf extraktivem Weg hergestellt werden können, und es ist bereits bekannt, derartige Zuckerlösungen bis zu Glucose abzubauen und eine auf diese Weise erhaltene Maische zu vergären. Der Gärprozeß verlangt hiebei je nach den eingesetzten Rohstoffen bzw. der eingesetzten Maische unterschiedliche Bedingungen und erfordert einen relativ langen Reaktionszeitraum für den Abschluß der Gärung. Nachteilig bei den bekannten Gärverfahren ist hiebei die Tatsache, daß die Maischen bzw. gärfähigen Substrate, im Verlauf der Gärung unterschiedliche Konzentrationen sowohl an vergärbarem Zucker als auch an Gärungsprodukten aufweisen, welche mit zunehmender Gärung die Reaktionsgeschwindigkeit bzw. die Gärungsgeschwindigkeit stark beeinflussen. Es ist weiters nachteilig, daß mit den bekannten Verfahren immer nur ein mehr oder minder reiner Alkohol und in der Regel mit geringer Konzentration hergestellt werden kann.

Die üblicherweise aus stärkehaltigen Rohstoffen durch Verzuckerung vergärbaren Zuckerlösungen weisen relativ geringe Zuckerkonzentrationen auf. Mit Rücksicht auf eine raschere Gärung ist es in diesen Fällen vorteilhaft, eine Volumensreduktion bei gleichzeitiger Erhöhung der Zuckerkonzentration vorzunehmen.

Aus der WO/03182 ist bereits ein Verfahren der eingangs genannten Art bekannt geworden, bei welchem der gebildete Alkohol zusammen mit dem gebildeten $CO_2$ während der Gärung abgesaugt wird. Bei diesem bekannten Verfahren wurde eine Zuckerlösung eingesetzt, deren Wassergehalt während des Verfahrens unter Berücksichtigung der über die Gasphase abgezogenen Wassermenge ergänzt wurde.

Die Erfindung zielt nun darauf ab, eine besonders einfache Verfahrensweise zu schaffen, mit welcher ein unmittelbar vergärbares Substrat mit einer für die nachfolgende Gärung günstigen Zuckerkonzentration geschaffen wird, und auf die apparativ und energetisch aufwendigen Eindickungsgeräte verzichtet werden kann. Zur Lösung dieser Aufgabe besteht die Erfindung im wesentlichen darin, daß der gebildete Hefeschlamm von der entstandenen Mischung abgetrennt wird, daß der Hefeschlamm in den Reaktionsraum eingebracht wird, daß die Hefe nach Abschluß des Gärungsprozesses aus dem Gärbehälter ausgetragen wird und nach einer Belüftung und nach Versetzen mit einer Nährstofflösung, insbesondere mit Kohlenstoffträgern und/oder Nährsalzen, zumindest teilweise der Zuckerlösung vor dem Eintragen in den Gärbehälter zur Herstellung der Mischung rückgeführt wird. Durch den Zusatz von Hefe wird nach einer Reaktionszeit von etwa 10 Minuten Glucose aus der Zuckerlösung von der Hefe aufgenommen. Der Einsatz des Hefeschlammes, mit welchem die Glucose angereichert ist, führt zu einer besonders raschen Vergärung des in der Zuckerlösung enthaltenen Zuckers und auf Grund der schonenden Verfahrensbedingungen kann nicht nur der Großteil der handelsüblichen Hefen bei guten Gärleistungen verwendet werden, sondern es ist auch ein Führen der Hefe im Kreislauf und damit eine besonders umweltschonende Verfahrensführung möglich. Zu diesem Zweck wird erfindungsgemäß die Hefe nach Abschluß des Gärprozesses aus dem Gärbehälter ausgetragen und nach einer Belüftung und nach Versetzen mit einer Nährstofflösung, insbesondere mit Kohlenstoffträgern und/oder Nährsalzen, zumindest teilweise der Zuckerlösung vor dem Eintragen in den Gärbehälter zur Herstellung der Mischung rückgeführt. Die überschüssige Hefe kann abgetrennt werden und einer anderen Verwendung zugeführt werden.

Die Hefe wird, da der gebildete Alkohol kontinuierlich bereits während der Gärung destillativ abgetrennt wird, nie oder nur kurzzeitig höheren Konzentrationen an Gärungsprodukten ausgesetzt, so daß eine bessere Vitalität des biologischen Materials vorliegt. Es ist im Rahmen des erfindungsgemäßen Verfahrens eine Regeneration der Hefe nur im geringen Umfang notwendig.

Die Abtrennung des Hefeschlammes aus der Mischung mit der Zuckerlösung kann in besonders vorteilhafter Weise durch Flotation erfolgen, wobei die bereits einsetzende Gasentwicklung ausgenutzt werden kann und eine Flockung durch Zusatz von Polyelektrolyten vorgesehen sein kann. Flockung, Flotation und Hefeabtrennung können bei Gärungstemperatur (30 bis 35 °C) erfolgen. Gegebenenfalls wird bereits die Mischung der Hefe mit der Glucoselösung bei Gärungstemperatur durchgeführt. Die Flockung kann mit Vorteil so durchgeführt werden, daß die vom Hefeschlamm vor dem Einbringen desselben in den Gärbehälter abgetrennte Lösung zusammen mit Polyelektrolyten der Flockung zugesetzt wird, wobei auf diese Weise die noch zuckerhaltige Lösung, welche vom Hefeschlamm abgetrennt wurde, zumindest teilweise im Kreislauf geführt werden kann. Eine Führung der Hefe im Kreislauf wird insbesondere durch die kurze Gärungsdauer ermöglicht. Zur Regeneration der Hefe wird diese nach dem Ausbringen aus dem Gärbehälter einer aeroben Phase unterworfen, wobei in regelmäßigen Abständen Hefe abgezogen werden kann und für die Futtermittelproduktion verwendet werden kann.

Dadurch, daß die Gärung unter einem unteratmosphärischen Durck bzw. Vakuum durchgeführt wird, wird während der Gärung die entstehende Kohlensäure ebenso wie das entstehende Äthanol aus dem Substrat abgeführt. Die Alkoholkonzentration nimmt somit im Substrat im Verlauf der Gärung nicht zu und es bleibt ein über den gesamten Verlauf der Gärung gleichbleibendes Milieu für die Gärung aufrecht, wodurch sich neben der verbesserten Vitalität der Hefe für die neuerliche Verwendung eine höhere Glucoseumsetzung ergibt. Erfindungsgemäß wird hierbei vorzugsweise während der Gärung ein Druck von 100 mbar bis 400 mbar, vorzugsweise 260 bis 330 mbar, im Gärbehälter aufrechterhalten, wobei die Gärung vorzugsweise in an sich bekannter Weise bei Temperaturen von 30 bis 35 °C durchgeführt wird.

Da erfindungsgemäß von einer vergärbaren Zuckerlösung ausgegangen wird, ist der Verlauf der Gärung und die Gärungsgeschwindigkeit in keiner Weise durch Verdünnungs- und Diffusionsvorgänge im Substrat beeinflußt und das Gärverfahren kann vom Beginn der Gärung bis zu dem relativ abrupt eintretenden Ende derselben bei weitgehend konstanter Gärgeschwindigkeit in wesentlicher kürzerer Zeitdauer durchgeführt werden, als dies bei üblichen Gärprozessen der Fall ist. Das Verfahren ist darüberhinaus in besonders einfacher Weise steuerbar, wobei lediglich physikalische Parameter, wie Druck und Temperatur, den jeweiligen Erfordernissen angepaßt werden müssen. Die für die Gärung zum Einsatz gelangenden Enzyme können beliebig aus den bekannten, zur Unterstützung der Äthanolgärung entwickelten Enzymen ausgewählt werden, da die Verfahrensbedingungen so schonend gewählt sind, daß keine Beeinträchtigung des Gärvorganges erfolgt.

Vorzugsweise wird erfindungsgemäß der Druck im Reaktionsraum zumindest gegen Ende der Fermentationsdauer zur Vervollständigung der destillativen Abtrennung des Äthanols, vorzugsweise auf 150 bis 300 mbar, weiter abgesenkt, wobei der Druck im Gärbehälter nach einer ersten Phase der Vergärung, insbesondere nach etwa einem Viertel der gesamten Fermentationsdauer abgesenkt werden kann.

Gemäß einer bevorzugten Weiterbildung des erfindungsgemäßen Verfahrens wird während des Gärvorganges, zumindest jedoch in der ersten Phase der Gärung, $CO_2$ durch die Zuckerlösung hindurchgeleitet, wobei für die Aufrechterhaltung des unteratmosphärischen Druckes Sorge getragen wird. Die gleichzeitige Anwendung eines Unterdruckes und das Hindurchleiten von $CO_2$ hat überraschenderweise gezeigt, daß die Gärungsbakterien die Hexosen schneller umsetzen und eine raschere Gärung erfolgt.

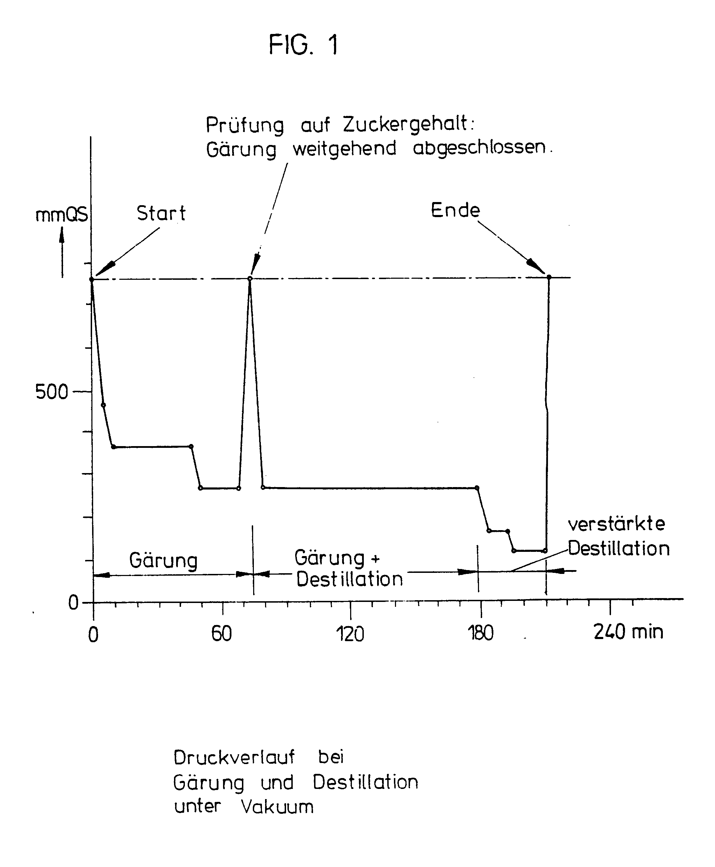

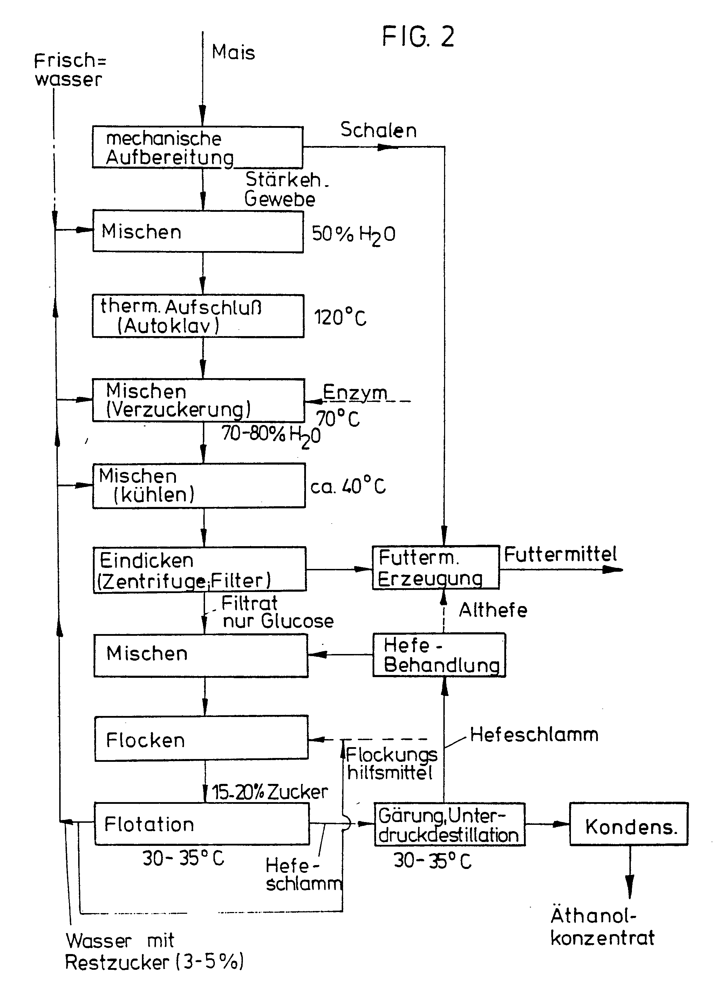

Die Erfindung wird nachfolgend an Hand der in der Zeichnung dargestellten Druckverlaufskurve und eines Ausführungsbeispieles näher erläutert. In dere Zeichnung zeigt Fig. 1 eine Druckverlaufkurve für das erfindungsgemäße Gärverfahren und Fig. 2 ein vereinfachtes Fließschema für eine vollständige Aufbereitung von stärkehaltigen Rohstoffen zu Alkohol.

Die angegebenen Mengen im Ausführungsbeispiel beziehen sich hiebei auf 1 l Zuckerlösung.

| | |
|---|---:|
| Glucoseeinsatz | 182,8 g |
| Hefeeinsatz | 1,47 kg Schlamm mit |
| | ca. 4 % TS |
| Reaktionszeit | 10 Minuten |
| Polyelektrolyteinsatz | 320 mg |
| | ($\hat{=}$ 1,75 g/kg Glucose) |
| Reaktionszeit | 10 Minuten |
| Flotationszeit | 7,0 Minuten |
| abgezogene Zuckerlösung | 1,10 l |
| abgezogener Restzucker | 52,9 g Glucose |
| Zucker mit Hefeschlamm zur Gärung | 129,9 g |

Die Gärung wurde analog der Darstellung der Zeichnungsfigur so durchgeführt, daß der Druck unmittelbar nach dem Start auf 350 mbar abgesenkt wurde. Nach etwa 45 Minuten wurde der Druck weiter auf 260 mbar abgesenkt und nach etwa 70 Minuten der Zuckergehalt bestimmt. Innerhalb dieser Zeit ist die Gärung bereits weitgehend abgeschlossen und es tritt bereits während dieser Zeit eine Destillation des entstehenden Alkohols ein. Nach etwa 180 Minuten wurde der Druck im Reaktionsraum weiter stufenweise abgesenkt, um eine vollständige Destillation zu erzielen und die Äthanolausbeute zu vervollständigen. Je 1 l Zuckerlösung wurden hiebei eine Menge von 0,34 l Destillat mit einem Äthanolgehalt von 0,077 l, das heißt, 60,7 g Äthanol, gewonnen. Die Ausbeute des adsorbierten Zuckers beträgt hiebei

$$60,7/(129,9 \cdot 0,511) = 0,915$$

Glucose konnte im Hefeschlamm nicht nachgewiesen werden.

Für die Durchführung des gesamten Verfahrens ergibt sich folgender Zeitablauf :

| | |
|---|---:|
| Glucoseadsorption | 0,17 h |
| Flockung | 0,02 h |
| Flotation | 0,12 h |
| Gärung + Destillation | 3,50 h |
| gesamte Prozeßdauer = | 3,81 h |

Die im Kreislauf geführte Lösung, welche vom Hefeschlamm vor dem Einbringen in den Reaktionsraum abgetrennt wurde, enthält durchschnittlich 3 bis 5 Gew.% Glucose.

Der Verfahrensablauf einer Aufbereitung von zuckerhaltigen Rohstoffen bis zum Äthanol ist schematisch in Fig. 2 dargestellt. Aus diesem Fließschema ergeben sich auch die erfindungsgemäß vorgeschlagenen Kreislaufführungen zum Zwecke der Verbesserung der Energieausbeute bzw. der Glucoseausbeute.

**Patentansprüche**

1. Verfahren zur Gewinnung von Äthanol aus vergärbaren Zuckerlösungen, bei welchem die Zuckerlösung vor dem Einbringen in einen Gärbehälter mit Hefe versetzt wird, und der durch Gärung gebildete Alkohol durch Anwendung von unteratmosphärischem Druck über die Gasphase aus dem Gärbehälter abgezogen wird, dadurch gekennzeichnet, daß der gebildete Hefeschlamm von der entstandenen Mischung abgetrennt wird, daß der Hefeschlamm in den Reaktionsraum eingebracht wird, daß die Hefe nach Abschluß des Gärprozesses aus dem Gärbehälter ausgetragen wird und nach einer Belüftung und nach Versetzen mit einer Nährstofflösung, insbesondere mit Kohlenstoffträgern und/oder Nährsalzen, zumindest teilweise der Zuckerlösung vor dem Eintragen in den Gärbehälter zur Herstellung der Mischung rückgeführt wird.

2. Verfahren nach Anspruch 1, dadurch gekennzeichnet, daß die Mischung vor dem Abtrennen des Hefeschlammes, beispielsweise durch Flotation, einer Flockung unterworfen wird.

3. Verfahren nach Anspruch 2, dadurch gekennzeichnet, daß die vom Hefeschlamm vor dem Einbringen desselben in den Gärbehälter abgetrennte Lösung zusammen mit Polyelektrolyten der Flockung zugesetzt wird.

4. Verfahren nach einem der Ansprüche 1 bis 3, dadurch gekennzeichnet, daß während der Gärung ein Druck von 100 mbar bis 400 mbar, vorzugsweise 260 bis 330 mbar, im Gärbehälter aufrechterhalten wird, wobei die Gärung vorzugsweise in an sich bekannter Weise bei Temperaturen von 30 bis 35 °C durchgeführt wird.

5. Verfahren nach einem der Ansprüche 1 bis 4, dadurch gekennzeichnet, daß der Druck im Gärbehälter zumindest gegen Ende der Fermentationsdauer zur Vervollständigung der destillativen Abtrennung des Äthanols vorzugsweise auf 150 bis 300 mbar abgesenkt wird.

6. Verfahren nach einem der Ansprüche 1, 2 oder 3, dadurch gekennzeichnet, daß der Druck im Gärbehälter nach einer ersten Phase der Vergärung, insbesondere nach etwa einem Viertel der gesamten Fermentationsdauer, weiter abgesenkt wird.

7. Verfahren nach einem der Ansprüche 1 bis 6, dadurch gekennzeichnet, daß während der Gärung $CO_2$ durch die Zuckerlösung hindurchgeleitet wird.

**Claims**

1. A process for obtaining ethanol from fermentable sugar solutions in which yeast is added to the sugar solution prior to introduction into a fermentation vessel and the alcohol formed by fermentation is drawn off from the fermentation vessel via the gas phase using sub-atmospheric pressure, characterised in that the yeast sludge formed is separated off from the resulting mixture, that the yeast sludge is introduced into the reaction space, that the yeast is removed from the fermentation vessel after the end of the fermentation process and, after it has been aerated and mixed with a nutrient solution, particularly with carbon carriers and/or nutrient salts, is recycled at least partially to the sugar solution prior to introduction into the reaction vessel to form the mixture.

2. A process according to claim 1, characterised in that the mixture undergoes flocculation, e. g. by flotation, before the yeast sludge is separated off.

3. A process according to claim 2, characterised in that the solution which is separated from the yeast sludge before the latter is introduced into the fermentation vessel is mixed together with the polyelectrolytes for flocculation.

4. A process according to any one of claims 1 to 3, characterised in that during the fermentation a pressure of 100 mbar to 400 mbar, preferably 260 to 330 mbar, is maintained in the fermentation vessel, the fermentation preferably being carried out in a manner known per se at temperatures of 30 to 35 °C.

5. A process according to any one of claims 1 to 4, characterised in that the pressure in the fermentation vessel is reduced, at least towards the end of the fermentation period, preferably to 150 to 300 mbar to complete the separation of the ethanol by distillation.

6. A process according to any one of claims 1, 2 or 3, characterised in that the pressure in the fermentation vessel is reduced further after the first phase of the fermentation, particularly after about a quarter of the whole period of fermentation.

7. A process according to any one of claims 1 to 6, characterised in that during the fermentation $CO_2$ is passed through the sugar solution.

# EP 0 114 161 B1

## Revendications

1. Procédé de préparation d'éthanol à partir de solutions de sucre fermentescibles, dans lequel la solution de sucre est additionnée de levure avant d'être introduite dans un récipient de fermentation, et l'alcool formé par fermentation, est extrait du récipient de fermentation par application d'une pression inférieure à la pression atmosphérique, par la phase gazeuse, caractérisé en ce que la boue de levure formée est séparée du mélange obtenu, en ce que la boue de levure est introduite dans l'enceinte de réaction, en ce que la levure est extraite du récipient de fermentation après achèvement du processus de fermentation, et est renvoyée, au moins partiellement, à la solution de sucre avant introduction dans le récipient de fermentation, après aérage et addition d'une solution nutritive, en particulier de supports de carbone et/ou de sels nutritifs, pour préparation du mélange.

2. Procédé selon la revendication 1, caractérisé en ce que le mélange est soumis à un floculation avant séparation de la boue de levure, par exemple par flottation.

3. Procédé selon la revendication 2, caractérisé en ce que la solution séparée de la boue de levure avant introduction de cette dernière dans le récipient de fermentation, est soumise à floculation avec des polyélectrolytes.

4. Procédé selon l'une des revendications 1 à 3, caractérisé en ce qu'une pression de 100 mbar à 400 mbar, de préférence entre 260 et 330 mbar, est maintenue dans le récipient de fermentation, pendant la fermentation, celle-ci se faisant de manière connue en soi, à des températures comprises entre 30 et 35 °C.

5. Procédé selon l'une des revendications 1 à 4, caractérisé en ce que la pression régnant dans le récipient de fermentation est abaissée à une pression comprise de préférence entre 150 et 300 mbar, au moins vers la fin de la durée de fermentation, pour compléter la séparation de l'éthanol par distillation.

6. Procédé selon l'une des revendications 1, 2 ou 3, caractérisé en ce que la pression régnant dans le récipient de fermentation, est encore abaissée après une première phase de fermentation, en particulier après environ un quart de la durée totale de fermentation.

7. Procédé selon l'une des revendications 1 à 6, caractérisé en ce que du $CO_2$ est envoyé à travers la solution de sucre, pendant la fermentation.

5

# FIG. 1

Druckverlauf bei
Gärung und Destillation
unter Vakuum

FIG. 2

Frisch=
wasser

Mais

mechanische
Aufbereitung

Schalen

Stärkeh.
Gewebe

Mischen — 50% $H_2O$

therm. Aufschluß
(Autoklav) — 120°C

Mischen
(Verzuckerung) ← Enzym
70°C
70-80% $H_2O$

Mischen
(kühlen) — ca. 40°C

Eindicken
(Zentrifuge,Filter) → Futterm.
Erzeugung → Futtermittel

Filtrat
nur Glucose

Althefe

Mischen ← Hefe -
Behandlung

Flocken

Hefeschlamm

Flockungs
hilfsmittel

15-20% Zucker

Flotation → Gärung,Unterdruckdestillation → Kondens.

30-35°C
Hefeschlamm
30-35°C

Äthanolkonzentrat

Wasser mit
Restzucker (3-5%)